(19) Europäisches Patentamt
European Patent Office
Office européen des brevets

(11) **EP 4 696 373 A1**

(12) **EUROPEAN PATENT APPLICATION**
published in accordance with Art. 153(4) EPC

(43) Date of publication:
**18.02.2026   Bulletin 2026/08**

(21) Application number: **24792022.6**

(22) Date of filing: **17.04.2024**

(51) International Patent Classification (IPC):
**A61N 5/10** (2006.01)

(52) Cooperative Patent Classification (CPC):
**A61N 5/10**

(86) International application number:
**PCT/CN2024/088210**

(87) International publication number:
**WO 2024/217437 (24.10.2024 Gazette 2024/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:   17.04.2023   CN 202310410669
16.04.2024   CN 202410458331

(72) Inventors:
• **TENG, Yi-chiao**
**Nanjing, Jiangsu 211112 (CN)**
• **CHEN, Jiang**
**Nanjing, Jiangsu 211112 (CN)**

(74) Representative: **Gunzelmann, Rainer**
**Wuesthoff & Wuesthoff**
**Patentanwälte und Rechtsanwalt PartG mbB**
**Schweigerstraße 2**
**81541 München (DE)**

(71) Applicant: **Neuboron Therapy System Ltd.**
**Xiamen, Fujian 361026 (CN)**

(54) **LOCALIZATION METHOD AND SYSTEM FOR REGION OF INTEREST IN RADIATION THERAPY**

(57)     A method and a system for locating a region of interest in radiation therapy are provided. The method includes: acquiring first image data of an irradiated body in a first state or a second state; acquiring second image data of the irradiated body in the second state; registering and aligning the first image data with the second image data and acquiring a reference region based on the first image data or the second image data; obtaining a marker parameter based on the second image data, the marker parameter of the reference region being a reference value; and determining a set value based on a reference value and defining a region of interest based on the set value.

FIG. 1

EP 4 696 373 A1

## Description

### TECHNICAL FIELD

The present application relates to the technical field of radiation therapy, and in particular to a method and a system for locating a region of interest in radiation therapy.

### BACKGROUND

With the development of atomic science, radiation therapy such as cobalt-60, linear accelerators and electron beams has become one of the principal means for cancer treatment. However, conventional photon or electron therapy is limited by the physical conditions of radiation itself and may cause damages to a large number of normal tissues in a beam path while killing tumor cells. Furthermore, due to differences in the sensitivity of the tumor cells to the radiation, the efficacy of the conventional radiation therapy is often unsatisfactory for malignant tumors with higher radioresistance. To reduce radiation damages to normal tissues around a tumor, the concept of targeted therapy from chemotherapy has been applied to radiation treatment. For the tumor cells with high radioresistance, currently, radiation sources with high relative biological effectiveness (RBE), such as proton therapy, heavy particle therapy, and neutron capture therapy, are actively developed. The neutron capture therapy combines both of the concepts mentioned above. For example, boron neutron capture therapy (BNCT) utilizes the specific accumulation of boron-containing drugs in tumor cells, in combination with precise beam control, to offer a superior cancer treatment option over the conventional radiation therapy.

During radiation therapy such as boron neutron capture therapy, a labeled boron-containing drug needs to be administered into an irradiated body and the drug will selectively accumulate in tumor cells, tissues and organs around the tumors, or other normal tissues and organs. Under neutron irradiation, the physical dose required to achieve the same biological effect varies among tissues and organs that have absorbed the boron-containing drug. The compound biological effectiveness (CBE) can effectively evaluate the biological response of the boron-containing drug to specific tissues under irradiation. A precise radiation treatment plan must be made to implement a highly conformal dose distribution in a gross tumor volume and reduce the radiation dose to an organ at risk (OAR) so as to reduce radiation-induced toxicity. This requires accurate segmentation of OAR on planned CT images, and the regions where the organs at risk are located are defined as regions of interest. In the prior art, the delineation of the regions of interest is typically performed manually by specialized physicians. This is often time-consuming and the result heavily depends on the expertise of the physicians, lacking guarantees of accuracy and consistency, which in turn affects the assessment of the irradiation dose in the treatment plan.

### SUMMARY

In view of this, in order to solve the above technical problems, it is necessary to provide a method and a system for locating a region of interest in radiation therapy, which allow to directly and accurately obtain a region of interest.

In a first aspect, the present application provides a method for locating a region of interest in radiation therapy. An irradiated body has a first state in which no marker is injected and a second state in which a marker is injected. The method comprising: acquiring first image data of the irradiated body in a first state or a second state; acquiring second image data of the irradiated body in the second state; registering and aligning the first image data with the second image data; acquiring a reference region based on the first image data or the second image data, where the reference region acquired based on the first image data has positional information consistent with that of the reference region acquired based on the second image data. obtaining a marker parameter based on the second image data, the marker parameter of the reference region being a reference value; and determining a set value based on the reference value and defining a region of interest based on the set value.

In one embodiment, the set value is a ratio of a marker parameter of a region to be defined to the reference value, and the set value is at least 1.5.

In one embodiment, the set value is at least 2.

In one embodiment, the set value is the marker parameter of a region to be defined, and the set value is at least 1.5 times the reference value.

In one embodiment, the marker parameter is at least one of marker radioactivity, marker radiation intensity, a number of atoms decaying per unit time, an effective count of annihilation photons, a semi-quantitative standardized uptake value, and a standardized uptake value.

In one embodiment, the marker parameter is a standardized uptake value, and the standardized uptake value is calculated by the following formula:

$$SUV_{ROI} = \frac{A_{C,ROI}}{D \times 2^{\left(-\Delta t / T_{\frac{1}{2}}\right)}} \times W$$

where $SUV_{ROI}$ is a standardized uptake value for a region of interest; $A_{C,ROI}$ is a drug radioactivity concentration in the region of interest; $W$ is a body weight of the irradiated body; $\Delta t$ is a delay between time of drug injection and time of scan start; $T_{\frac{1}{2}}$ is a half-life of a drug's radionuclide; and $D$ is a dose of an injected drug.

In one embodiment, the region of interest is defined in the first image data based on the set value.

In one embodiment, a region in the second image data where the marker parameter conforms to the set value are mapped to the first image data and a region of interest is defined in the first image data.

In one embodiment, the second image data is obtained by fusing a second functional image with a second sectional image.

In one embodiment, the first image data is registered with data of the second sectional image, and the first image data has a higher definition than the data of the second sectional image.

In one embodiment, the first image data is acquired by a first imaging device, and the second image data is acquired by a second imaging device different from the first imaging device.

In one embodiment, the second imaging device is a multi-mode imaging device.

In one embodiment, emission computed tomography and computed tomography are used for the multi-mode imaging device.

In a second aspect, the present application further provides a system for locating a region of interest in radiation therapy. An irradiated body has a first state in which no marker is injected and a second state in which a marker is injected. the system comprising: an imaging device configured to acquire first image data of the irradiated body in the first state or the second state, and second image data of the irradiated body in the second state; and a processing device configured to: acquire a reference region based on the first image data or the second image data, the reference region acquired based on the first image data having positional information consistent with that of the reference region acquired based on the second image data; obtain a marker parameter based on the second image data, the marker parameter of the reference region being a reference value; and determine a set value based on the reference value and define a region of interest based on the set value.

In one embodiment, the set value is a ratio of a marker parameter of a region to be defined to the reference value, and the set value is at least 1.5.

In one embodiment, the set value is the marker parameter of a region to be defined, and the set value is at least 1.5 times the reference value.

In the method and the system for locating a region of interest in radiation therapy, the reference region is determined based on the first image data, and the reference region is mapped to the second image data, and the set value is determined using the marker parameter of the reference region in the second image data, so as to determine the region of interest of the irradiated body based on the set value. In this way, the region of interest can be quickly and accurately delineated, irradiation conditions for a patient can be correctly evaluated, the accuracy and reliability of a treatment plan in evaluating a normal tissue tolerance dose or a tumor prescription dose can be improved, and serious side effects caused by excessive exposure of the normal tissues of the irradiated body to the radiation can be avoided.

**BRIEF DESCRIPTION OF THE DRAWINGS**

FIG. 1 is a schematic flowchart of a method for locating a region of interest according to an embodiment;
FIG. 2 is a schematic structural diagram of a BNCT system according to an embodiment;
FIG. 3 is a schematic structural diagram of a system for locating a region of interest according to an embodiment;
FIG. 4 shows an example of first image data according to an embodiment;
FIG. 5 shows an example of second image data according to an embodiment, where (1) is an example of a second functional image, (2) is an example of a second sectional image, and (3) is an example of an image formed by fusing the second functional image with the second sectional image;
FIG. 6(1) shows an example of delineating a region of interest in FIG. 4 based on a marker accumulation region shown in (3) of FIG. 5; and
FIG. 6(2) shows an example of delineating a region of interest adjacent to a tumor based on a method according to an embodiment of the present invention.

List of reference numerals:

BNCT system: 100; Radiation generation device: 110; Accelerator: 111; Beam transmission device: 112; Beam shaping assembly: 120; Collimator: 126; Reflector: 121; Moderator: 122; Thermal neutron absorber: 123; Radiation shield: 124; Beam channel: 125;
Localization system: 200; Processing device: 210; Imaging device: 220; First imaging device: 221; Second imaging device: 222; Computation module: 211; Storage module: 212; Communication module: 213.

## DETAILED DESCRIPTION OF THE EMBODIMENTS

In order to make the objective, technical solutions, and advantages of the present application clearer, the present application is further described in detail below with reference to the accompanying drawings and the embodiments. It should be understood that the specific embodiments described herein are only for the purpose of explaining rather than limiting the present invention.

The present embodiment provides a method for locating a region of interest in radiation therapy. Radiation therapy is a treatment method in which radioactive irradiation is delivered to an irradiated body. The irradiated body includes a region of interest and a reference region. The region of interest is a region with relatively high sensitivity to the radioactive irradiation. The reference region is a region that can be definitely delineated and confirmed based on first image data. The irradiated body further includes a tumor region requiring irradiation therapy. The tumor region includes a tumor or a cancer tissue. The irradiated body has a first state in which no marker is injected and a second state in which a marker is injected. The method for injecting a marker may include intravenous injection, input to the digestive system, etc.

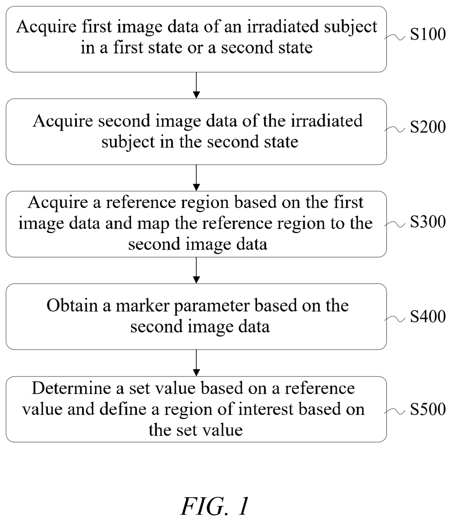

As shown in FIG. 1, the present embodiment provides a method for locating a region of interest in radiation therapy. The method specifically includes the following steps:

S100: acquiring first image data of an irradiated body in a first state or a second state;
S200: acquiring second image data of the irradiated body in the second state;
S300: acquiring a reference region based on the first image data and mapping the reference region to the second image data;
S400: obtaining a marker parameter based on the second image data, the marker parameter of the reference region being a reference value; and
S500: determining a set value based on the reference value and defining a region of interest based on the set value.

In the method for locating a region of interest in the present embodiment, the second image data from which the marker parameter can be read and the first image data from which the reference region can be identified are used in combination to define a set value based on the marker parameter of the reference region, and the region of interest of the irradiated body is determined based on the set value. In this way, the region of interest can be quickly and accurately delineated, irradiation conditions for a patient can be evaluated properly, the accuracy and reliability of a treatment plan in evaluating a normal tissue tolerance dose or a tumor prescription dose can be improved, and serious side effects caused by excessive exposure of the normal tissues of the irradiated body to the radiation, which may affect the patient's recovery or quality of life, can be prevented.

In the present embodiment, the boron neutron capture therapy (BNCT) is preferred as the radiation therapy. The main principles of the boron neutron capture therapy are as follows: after a boron-containing ($^{10}$B) drug is administered to or injected into the irradiated body, the boron-containing drug selectively accumulates in tumor cells, then by virtue of the property of a high capture cross-section of the boron-containing ($^{10}$B) drug for thermal neutrons, two heavy charged particles, $^4$He and $^7$Li, are generated by means of $^{10}$B(n, $\alpha$)$^7$Li neutron capture and nuclear fission reaction, the two heavy charged particles have an average energy of about 2.33 MeV and feature high linear energy transfer (LET) and a short range, the total range of the two particles is approximately equal to the size of one cell, and the radiation damage caused to an organism can be thus confined to the cellular level, such that the purpose of locally killing tumor cells without causing excessive damage to normal tissues can be achieved. By means of the specific accumulation of the boron-containing drug in tumor cells, in combination with the precise control of a neutron beam, a superior cancer treatment option over conventional radiation is provided.

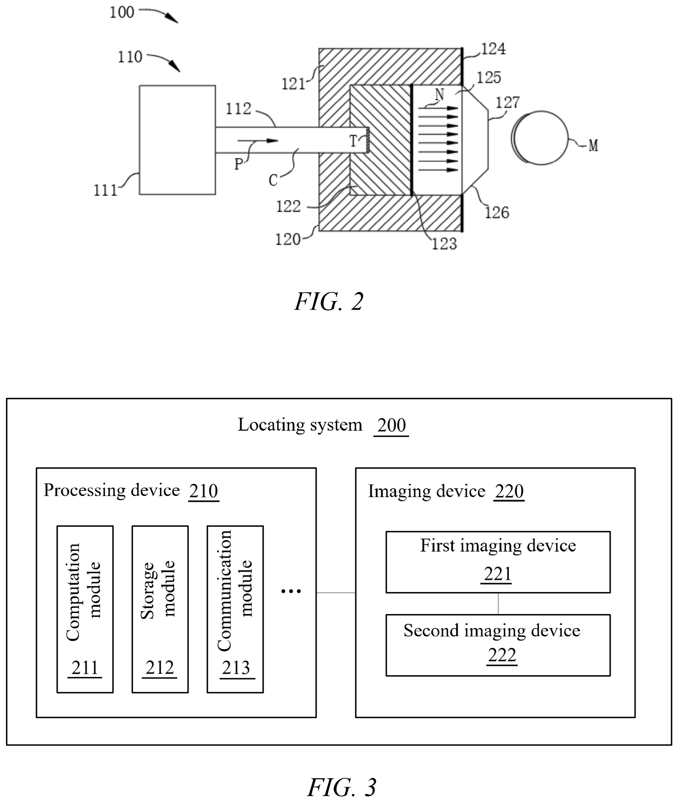

As shown in FIG. 2, a BNCT system 100 includes a radiation generation device 110 and a beam shaping assembly 120. The radiation generation device 110 includes an accelerator 111, a beam transmission device 112, and a target T. The accelerator 111 accelerates charged particles (such as protons and deuterons) to produce a charged particle beam P such as a proton beam. The beam transmission device 112 includes a beam transmission tube C. The charged particle beam P is transmitted through the beam transmission tube C, irradiates the target T and interacts with the target T to produce a neutron beam N. The target T is preferably a metal target. A suitable nuclear reaction is selected based on properties such as the required neutron yield and energy, available accelerated charged particle energy and current magnitude, and the physical and chemical properties of the metal target. Nuclear reactions that are often discussed include $^7Li(p,n)^7Be$ and $^9Be(p, n)^9B$, both of which are endothermic reactions. The two nuclear reactions have respective energy thresholds of 1.881 MeV and 2.055 MeV. Since the ideal neutron source for the boron neutron capture therapy provides epithermal neutrons in the keV energy range, theoretically, if a lithium metal target is bombarded by protons having an energy only slightly above the thresholds, neutrons of relatively low energy can be produced. These neutrons could be used clinically without requiring extensive moderation. However, the cross-sections for the interaction of both lithium (Li) and beryllium (Be) metal targets with protons at threshold energy are not high. In order to create a sufficiently large neutron flux, protons with high energy are typically selected to induce the nuclear reaction. An ideal target should have properties such as a high neutron yield, a generated neutron energy distribution close to the epithermal neutron energy range, minimal production of intense penetrating radiation, safety, low cost, ease of handling, and high-temperature resistance. However, in practice, it is impossible to find a nuclear reaction that meets all these requirements. In the embodiments of the present invention, a target made of lithium metal is preferred. However, as is well-known to those skilled in the art, the target may also be made of a metal material other than lithium or beryllium. For example, the target may be formed of tantalum (Ta) or tungsten (W). The target may be disk-shaped, may have another solid shape, or may use a liquid material (liquid metal).

A collimator 126 for converging a neutron beam is provided at an end of the beam shaping assembly 120 close to the irradiated body M. The beam shaping assembly 120 adjusts the beam quality of the neutron beam. The neutron beam N generated by the radiation generation device 20 sequentially passes through the neutron beam of the beam shaping assembly 120, and then irradiates the irradiated body M from a beam exit 127. The collimator 126 is configured to converge the neutron beam to endow the neutron beam with high targeting ability during treatment. It may be understood that, in the present embodiment, no collimator 126 may be provided, and the beam exits the beam shaping assembly 120 and then directly irradiates the irradiated body M.

The beam shaping assembly 120 further includes a reflector 121, a moderator 122, a thermal neutron absorber 123, a radiation shield 124, and a beam channel 125. Since the neutrons generated by the radiation generation device 110 have a broad energy spectrum, the contents of other types of neutrons and photons than the epithermal neutrons which meet therapeutic requirements need to be reduced as much as possible to prevent harm to an operator or the irradiated body. Therefore, neutrons coming from the target T need to pass through the moderator 122 to adjust the energy of fast neutrons of the neutrons (> 10 keV) within the epithermal neutron energy range (0.5 eV - 10 keV) and to reduce thermal neutrons (< 0.5 eV) as much as possible. The moderator 122 is made of a material having a high cross-section for interaction with fast neutrons and a low cross-section for interaction with epithermal neutrons. In a preferred embodiment, the moderator 122 is made of at least one of $D_2O$, $AlF_3$, Fluental™, $CaF_2$, $Li_2CO_3$, $MgF_2$, and $Al_2O_3$. The reflector 121 surrounds the moderator 122 and reflects the neutrons, which pass through the moderator 122 and diffuse in all directions, back into the neutron beam N to improve the utilization of the neutrons, and is made of a material having strong neutron reflection capability. In a preferred embodiment, the reflector 121 is made of at least one of Pb or Ni. The thermal neutron absorber 123 is located downstream of the moderator 122 and is made of a material having a high cross-section for interaction with thermal neutrons. In a preferred embodiment, the thermal neutron absorber 123 is made of Li-6. The thermal neutron absorber 121 is configured to absorb the thermal neutrons passing through the moderator 122 to reduce the content of the thermal neutrons in the neutron beam N, thereby preventing the exposure of superficial normal tissues to an excess dose during treatment. It may be understood that the thermal neutron absorber 123 may also be integral with the moderator whose material contains Li-6. The radiation shield 124 is configured to shield the neutrons and photons leaking from other parts than the beam channel 125. A material of the radiation shield 124 includes at least one of a photon shielding material and a neutron shielding material. In a preferred embodiment, the material of the radiation shield 124 includes plumbum (Pb) as the photon shielding material and polyethylene (PE) as the neutron shielding material. The beam exit 127 is disposed downstream of the beam channel 125. An epithermal neutron beam coming from the beam exit 127 radiates the irradiated body, is moderated into thermal neutrons after passing through the superficial normal tissues, and then reaches the tumor cells in the irradiated body M.

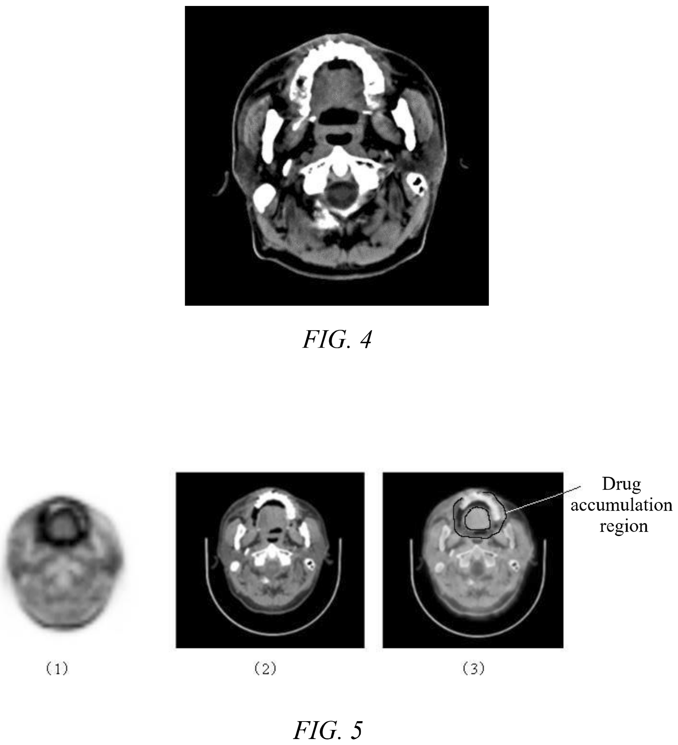

In step S100, the irradiated body in the first state or the second state is scanned by an imaging device and the first image data thereof is obtained. Further, in the present embodiment, the first image data is acquired by a first imaging device. The first imaging device is a computed tomography (CT) device or a magnetic resonance (MR) device, and may specifically be a normal CT device, an enhanced CT device, a CTsim device or an MR device. In the present embodiment, CTsim may particularly be used for the first imaging device. Compared to other image acquisition devices, an image with a higher definition is obtained by means of CTsim, thereby improving the accuracy of image alignment and region delineation in subsequent steps, as shown in FIG. 4. Because the first image data cannot show the information data of the markers, the marker or no marker may have been injected into the irradiated body when the first image data is acquired, that is, the irradiated body may be in either the first state or the second state in step S100. Further, there is no sequential relationship between step S100 and step S200 in terms of order.

In step S200, the irradiated body in the second state is scanned by an imaging device and the second image data thereof is obtained. The second image data is acquired by a radionuclide medical image acquisition apparatus and includes information on the marker parameter. Further, as shown in FIG. 5, in the present embodiment, the second image data is acquired by a second imaging device different from the first imaging device. The second imaging device is a multi-mode imaging device including at least a first-mode unit and a second-mode unit. The first-mode unit can acquire a second functional image containing image data of the marker parameter, and an example of the second functional image is shown in FIG. 5(1). The second-mode unit can acquire a second sectional image, and an example of the second sectional image is shown in FIG. 5(2). One of the first-mode unit and the second-mode unit can acquire or generate a transformation matrix for the second functional image and the second sectional image. The transformation matrix enables registration and fusion of the second functional image with the second sectional image. Further, the images imaged by the same or different methods are transformed utilizing the transformation matrix such that their spatial positions and spatial coordinates are matched. Spatial registration of the two images is performed by utilizing characteristics of their respective imaging methods, and the registered image data is fused into a single image. An example of the second image data obtained by means of fusion is shown in FIG. 5(3). In other embodiments, the multi-mode imaging device may further include an alignment and fusion unit for acquiring or generating a transformation matrix for the second functional image and the second sectional image and fusing the two images.

The multi-mode imaging device may specifically be a combination of an emission computed tomography (ECT) device and another imaging device, for example: a PET-CT device combining a positron emission tomography (PET) device and a CT device; an SPECT-CT device combining a single-photon emission computed tomography (SPECT) device and a CT device; or a PET-MR device, etc. Further, the second functional image is acquired by the ECT device, the second sectional image is acquired by a CT device, an MR device or the like, and the two images are fused. Further, In the case of PET-CT, PET-CT hybrid image fusion has the same positioning coordinate system. During scanning, it is not necessary to change the position of the irradiated body to perform PET-CT hybrid acquisition, which avoids errors caused by the displacement of the irradiated body and, to a certain extent, resolves registration issues in time and space, such that the image reliability is significantly improved. During a PET imaging process, due to attenuation factors such as annihilation reaction, scattering, random coincidence events and dead time, the acquired data is inconsistent with the actual situation, resulting in distorted image quality. Effective measures must be taken for correction. The multi-mode imaging device can perform attenuation correction on PET images using CT images or the like, thereby improving the usability of the PET images. In addition, since anatomical locations cannot be accurately identified in the PET images, fusion of image data is required.

In other embodiments, the first imaging device and the second imaging device may also be integrated in one and the same imaging device, which is a combination of an ECT device and another imaging device, for example, a PET-CT device, an SPECT-CT device, a PET-MR device, or the like. The second image data may be ECT functional image data, and the first image data may be sectional anatomical image data acquired by a CT device, an MR device, or the like. In other embodiments, the first imaging device may also be a CT device, an MR device, or the like, and the second imaging device may be an ECT device.

Before acquiring the second image data in step S200, the irradiated body has reached the second state, i.e., a relevant drug has been injected into the irradiated body as a marker, for example, a boron-containing ($^{10}$B) drug. The second image data may show a relevant parameter of the marker injected into a human body. The second image data may be a radionuclide image or image data based on a radionuclide image. The radioactive marker enters the human body and, after metabolism, creates a difference in radioactive concentration between the inside and outside of organs or between lesion sites and normal tissues. The second imaging device detects the difference. The second imaging device can dynamically observe the distribution and the changing process of the marker within the body, such as the conditions of functional metabolism in local tissues and organs. With the aid of an appropriate physiological mathematical model and software, a radioactive concentration distribution map of the image data can be converted into a parametric image of the marker. The boron-containing ($^{10}$B) drug containing a radioactive marker is injected into the irradiated body before irradiation therapy. Information related to boron ($^{10}$B) concentration can be obtained by means of the second image data. Further, the radioactive marker may be $^{18}$F. An $^{18}$F-labeled boron-containing drug injected into the irradiated body is absorbed by the cells of the patient, and positrons are emitted and immediately undergo annihilation with electrons within the irradiated body. Upon combination of a positron and an electron, two photons with identical energy and in opposite directions are released. This is a process known as positron annihilation. When both of this pair of photons are detected by a detector of the imaging device, it is counted as a valid event. Further, if the radioactively labeled boron-containing drug taken by the irradiated body is $^{18}$F-BPA, the second functional image is an $^{18}$F-BPA-PET image. It may be understood that $^{18}$F-BPA may also be replaced by other boron-containing drugs labeled with other radioactive markers or $^{18}$F, or radiolabeled non-boron-containing drugs with tumor cell affinity similar to that of boron-containing drugs, such as $^{18}$F-FDG.

In step S300, the positional information of the reference region is obtained from the first image data and the second image data. Prior to acquisition of the reference region, a corresponding type may be pre-selected. Based on the type, the reference region is defined directly in the first image data. The reference region should be a region that can be clearly identified and delineated in the anatomical image. In the present embodiment, the reference region includes normal soft tissues at a cardiac location or remote from a tumor. In the present embodiment, when step S100 is performed prior to step S200, the positional information of the reference region can be determined based on the first image data after step S100 is completed.

In the present embodiment, rigid registration, deformable registration, or a combination thereof may be used for the registration and alignment of the first image data and the second image data, so as to align the body contour ranges of the first image data and the second image data as closely as possible.

The registration and alignment of the first image data and the second image data specifically includes the following steps.

In step S311, a second functional image and a second sectional image are obtained. The second functional image and the second sectional image are separately obtained by the multi-mode imaging device.

In step S312, the second image data is obtained by fusing the second functional image with the second sectional image. In practical operations, in step S311 and step S312, a registered and fused PETCT image, i.e., the second image data, may be output in one step by a combined positron emission tomography and X-ray computed tomography (PET-CT) system.

In step S313, the first image data is registered with the second sectional image, and a transformation matrix is obtained. In the present embodiment, both the first image data and the second sectional image are obtained by the same imaging device, which can be a CT device or an MR device. For the image data obtained by the same imaging device, registration is easier to achieve. The imaging device may have a registration function and internally generate a transformation matrix from the first image data to the second sectional image. In addition, during the registration of the first image data and the second image data, because the second image data is obtained by fusion by means of the multi-mode imaging device, and the second functional image has been registered and fused with the second sectional image, upon that the first image data is registered with the second sectional image, the first image data can be registered and fused with the second image data. The registration process is accurate and quick, facilitating subsequent delineation of a region of interest and making of a treatment plan, so as to accurately and safely evaluate the radiation dose received by the region of interest and reduce the degree and probability of potential side effects.

A reference region can be acquired based on the second sectional image in the second image data. This reference region has positional information consistent with that of the reference region selected in the first image data.

In other embodiments, when both the first image data and the second image data are acquired by the same multi-mode imaging device, or when the first image data may be acquired by a CT device, an MR device , or the like, and the second image data may be acquired by an ECT device, step S300 may further include the following step S320: obtaining third image data by fusing the first image data with the second image data. It is noted that, during the fusion of the image data, registration needs to be performed on the image data first, but the registration step may not necessarily include an image fusion step.

In steps S200 and S300, the second image data based on the radionuclide medical image is acquired, and the marker parameter of the reference region of the second image data has been obtained. In step S400, the marker parameter shown after injecting the marker into the irradiated body are obtained based on the second image data and the marker parameter of the reference region of the second image data are taken as a reference value. In step S500, a set value is determined based on the above-mentioned reference value and a region of interest is defined in the second image data or the first image data based on the set value. The set value and the reference value are the marker parameters of different regions.

In general radiation therapies, regions of interest (ROIs) are different regions delineated by anatomical locations. In addition, during the making of a treatment plan and the determination of actual irradiation parameters, a biological equivalent dose (BED) needs to be determined based on a CBE value. Particularly, the BED is calculated by multiplying the physical dose values of individual components by respective RBE and/or CBE values and then summing the products.

In the early days, due to limited measurement means, it was considered in the BNCT that the boron concentration was the same for all normal tissues and blood. Compound biological effectiveness (CBE) values for various regions were obtained by means of biological experiments. For example, the CBE value for a mucosal tissue region as a region of interest was 4.9, the CBE value for the skin as a reference region was 2.5, and the CBE value for the brain was 1.3. With the development of measurement techniques, the concentration of a boron-containing drug in a specific region can be measured now. According to measurements, the concentration of the boron-containing drug in the mucosal tissue is approximately twice that in blood, and then the result of the calculation is corrected, resulting in a CBE value of 2.5 for the mucosal tissue. In the two cases described above, the results of the calculated biological equivalent dose (BED) are similar, but the results of the evaluation of the physical dose differ significantly. The higher the evaluated physical dose is, the easier it is to reach a dose limit, increasing the risk of exceeding the normal tissue tolerance dose. Mucositis is a common side effect in the BNCT. It can be seen from this that the delineation of an ORA (Organ at Risk, OAR) is of significant importance for making a radiation treatment plan.

The drug containing the marker accumulates in the tumor region to endow the tumor region with high sensitivity to the beam, and the tumor can be destroyed effectively during irradiation. The region of interest defined in the present embodiment specifically includes a non-tumorous region falling within the field of radiation in radiation therapy that exhibit relatively high sensitivity to the beam. Due to the physiological properties of the region of interest and other reasons, the drug containing the marker also accumulates to a considerable extent in the region of interest. However, the region of interest cannot be exposed to high doses of irradiation, making it necessary to accurately delineate the region of interest for subsequent dose evaluation. For example, if the tumor region contains an oral tumor, the region of interest includes an oral mucosal tissue. When inaccurate delineation of the region of interest leads to making an inaccurate treatment plan, the physical irradiation dose received by the mucosal tissue may reach or exceed a maximum physical dose of 6 Gy that is recommended internationally, which can easily induce grade 3 mucositis and thus affect the life and health of the irradiated body. Further, in the present embodiment, the region of interest may include other OAR regions close to the tumor region, or sensitive regions at a distance from the tumor region, such as those in a beam irradiation path that exhibit relatively high sensitivity to the beam. For both the OAR regions and the sensitive regions, accurate delineation is necessary.

The marker parameter refers to a parameter capable of indicating the differential expression of the marker in different regions. In the present embodiment, the marker parameter is at least one of marker radioactivity, marker radiation intensity, a number of atoms decaying per unit time, an effective count of annihilation photons, a semi-quantitative standardized uptake value, and a standardized uptake value. The numerical values presented in the PET image are photon count rates, and may relatively represent the radioactivity concentration Ac of the taken-up drug. The semi-quantitative parameter standardized uptake value (SUV) for a ROI in a particular tissue range is calculated by formula (1):

$$SUV_{ROI} = \frac{A_{C,ROI}}{D \times 2^{\left(-\Delta t / T_{\frac{1}{2}}\right)}} \times W \qquad (1)$$

where $SUV_{ROI}$ is a standardized uptake value for a region of interest; $A_{C,ROI}$ is a drug radioactivity concentration in the region of interest; $W$ is a body weight of the irradiated body; $\Delta t$ is a delay between time of drug injection and time of scan start; $T_{\frac{1}{2}}$ is a half-life of a drug's radionuclide; and $D$ is a dose of an injected drug.

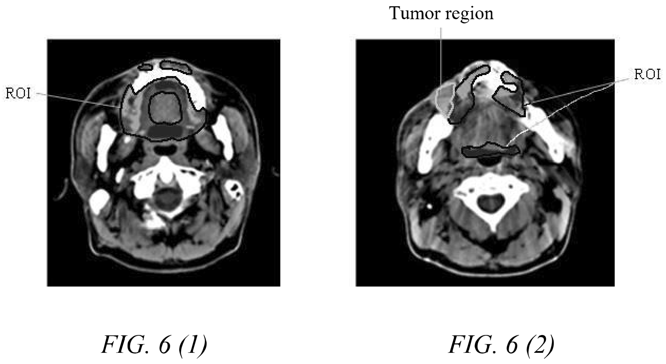

In step S400, the SUV value for the reference region is taken as a reference value, and in step S500, a set value is determined based on the SUV reference value. The set value, which may be data to be acquired and processed subsequently by the imaging device, may be input by a medical professional. In the present embodiment, the set value is a ratio of the marker parameter of the region of interest (ROI) to the reference value. When the region of interest is a mucosal region, the ratio is at least 1.5. As preferred in the present embodiment, the set value is at least 2. When the set value is within the above-mentioned value range, the region of interest may be delineated accurately and quickly in the first image data and the second image data. In other embodiments, the set value may also be a marker parameter of a region of interest, and a ratio of the set value to the reference value is at least 1.5. In conventional methods, a physician approximately delineates a region of interest in a CT image based on theoretical knowledge and common sense. This is dependent on the individual expertise of the physician, lacks accuracy and consistency, and may result in a deviation from a desired region and thus a failure to evaluate the dose distribution effectively. The region of interest shown in FIG. 6(1) is a region of interest, i.e., a mucosal region, that is delineated in a CTsim image of FIG. 4 based on a marker accumulation region in FIG. 5(3). The mucosal region as delineated in FIG. 6(1) includes a soft tissue and a bone tissue, still exhibiting a discrepancy from the determination of anatomical positions. In the method of the present application for delineates a region of interest based on a reference value, as shown in FIG. 6(2), a region of interest adjacent to a tumor region is delineated on FIG. 4 based on that the marker parameter in FIG. 5 is twice the marker parameter of the reference region, so as to make a more accurate and safer treatment plan.

It should be understood that although the steps in the flowchart involved in the embodiments as described above are shown sequentially as indicated by the arrows, the steps are not necessarily performed in the order indicated by the arrows. Unless explicitly stated herein, the order, in which these steps are performed, is not strictly limited, and the steps may be performed in other orders. Moreover, at least some of the steps in the flowchart involved in the embodiments as described above may include a plurality steps or stages. The steps or stages are not necessarily performed at the same time and instead they may be performed at different times. These steps or stages are not necessarily performed in a sequential order, and instead they may be performed in turn or alternately with other steps or at least some of the steps or stages of other steps.

On the basis of the same inventive concept, the present application further provides a system for locating a region of interest in radiation therapy. The solution provided by the device to address problems is analogous to the solution described in the method above. Therefore, for the specific definitions in one or more embodiments of the system for locating a region of interest in radiation therapy provided hereafter, reference may be made to the definitions for the above-described method for locating a region of interest in radiation therapy, which will be repeated here.

As shown in FIG. 3, a locating system 200 includes a processing device 210 and an imaging device 220. The imaging device 220 is configured to acquire first image data of an irradiated body in a first state or a second state, and second image data of the irradiated body in the second state. The processing device 210 is configured to: register and fuse the first image data with the second image data, and acquire a reference region from the first image data and the second image data; obtain a marker parameter based on the second image data; and determine a set value based on a reference value and define a region of interest based on the set value.

The imaging device 220 includes a first imaging device 221 and a second imaging device 222. The imaging device 220 may be a device integrating both the first imaging device 221 and the second imaging device 222, or a general term for the first imaging device 221 and the second imaging device 222 which are independent of each other. In the present embodiment, the first imaging device 221 is a computed tomography device, an X-ray imaging device or a magnetic resonance device, and particularly a common CT device, an enhanced CT device, a CTsim device or an MR device. The second imaging device 222 is a multi-mode imaging device, and may specifically be a device as a combination of an emission computed tomography device and another imaging device, such as a PET-CT device or a PET-MR device. The second imaging device 222 may acquire image data by fusing a second functional image with a second sectional image. The multi-mode imaging device may automatically perform image fusion, or image fusion may be achieved by an apparatus external to the localization system 200, such as an image fusion module or a treatment planning module. Data may be transmitted between the first imaging device 221 and the second imaging device 222 to complete image registration and fusion in step S300. The transmission method is not limited to wired or wireless transmission.

The processing device 210 includes at least a computation module 211, a storage module 212, and a communication module 213, and may further include a display module and/or an input module. In the present embodiment, the communication module 213 may acquire and receive the image data in the above steps S100 and S200, and the computation module 211 may perform steps S300, S400 and S500 to achieve automatic selection of the reference region, automatic registration of the first image data and the second image data, and automatic delineation of the region of interest. In other embodiments, the processing device 210 may also perform at least the steps of acquiring a reference region based on the first image data and the second image data and acquiring a marker parameter based on the second image data, and may perform the remaining steps in conjunction with inputs from external users.

All or some of the devices or modules of the localization system 200 described above may be implemented by software, hardware, or any combination thereof. The devices or modules described above may be embedded in or independent of a processor of a computer device in a form of hardware, or may be stored in a memory of a computer device in a form of software, such that the processor can invoke the modules to perform the operations corresponding to the modules.

Those skilled in the art can understand that the structure shown in FIG. 3 is only a block diagram of a partial structure related to the solution of the present application, and does not form a limitation on the computer device to which the solution of the present application is applied. The computer device may specifically include more or fewer components than those shown in the figure, have some components combined or have a different arrangement of the components.

It may be understood by those of ordinary skill in the art that all or some of the processes of the method of the previous embodiments may be completed by associated hardware instructed by a computer program. The computer program may be stored in a non-volatile computer-readable storage medium. When the computer program is executed, the processes in each previous method embodiment may be included. As provided in the embodiments, any reference to a storage module or any other medium may include at least one of a non-volatile memory and a volatile memory. The non-volatile memory may include a read-only memory (ROM), a magnetic tape, a floppy disk, a flash memory, an optical memory, a high-density embedded non-volatile memory, a resistive random access memory (ReRAM), a magnetoresistive random access memory (MRAM), a ferroelectric random access memory (FRAM), a phase change memory (PCM), a graphene memory, and the like. The volatile memory may include a random access memory (RAM), an external cache memory, and the like. By way of illustration rather than limitation, the RAM may be in various forms, such as a static random access memory (SRAM) or a dynamic random access memory (DRAM). The computation module involved in the embodiments provided in the present application may be, but is not limited to, a general-purpose processor, a central processing unit, a graphics processor, a digital signal processor, a programmable logic device, a quantum computing-based data processing logic device, and the like.

The technical features in the above embodiments can be arbitrarily combined. For brevity of description, not all possible combinations of the technical features in the above embodiments are described. However, any combination of these technical features without contradiction shall be considered to fall within the scope of the present specification.

The embodiments described above merely illustrate several implementations of the present application and are described relatively specifically and in detail, but should not be construed as limiting the patent scope of the present application. It should be noted that several variations and improvements may also be made by those of ordinary skill in the art without departing from the concept of the present application, and those modifications and improvements shall all fall within the scope of protection of the present application. Therefore, the scope of protection of the present application shall be defined by the appended claims.

**Claims**

1. A method for locating a region of interest in radiation therapy, **characterized in that** an irradiated body has a first state in which no marker is injected and a second state in which a marker is injected; the method comprising:

   acquiring first image data of the irradiated body in a first state or a second state;
   acquiring second image data of the irradiated body in the second state; registering and aligning the first image data with the second image data and acquiring a reference region based on the first image data or the second image data;
   obtaining a marker parameter based on the second image data, the marker parameter of the reference region being a reference value; and
   determining a set value based on the reference value and defining a region of interest based on the set value.

2. The method for locating a region of interest in radiation therapy according to claim 1, **characterized in that** the set value is a ratio of a marker parameter of a region to be defined to the reference value, and the set value is at least 1.5.

3. The method for locating a region of interest in radiation therapy according to claim 2, **characterized in that** the set value is at least 2.

4. The method for locating a region of interest in radiation therapy according to claim 1, **characterized in that** the set value is a marker parameter of a region to be defined, and the set value is at least 1.5 times the reference value.

5. The method for locating a region of interest in radiation therapy according to claim 1, **characterized in that** the marker parameter is at least one of marker radioactivity, marker radiation intensity, a number of atoms decaying per unit time, an effective count of annihilation photons, a semi-quantitative standardized uptake value, and a standardized uptake value.

6. The method for locating a region of interest in radiation therapy according to claim 4, **characterized in that** the marker parameter is a standardized uptake value, and the standardized uptake value is calculated by the following formula:

$$SUV_{ROI} = \frac{A_{C,ROI}}{D \times 2^{\left(-\Delta t / T_{\frac{1}{2}}\right)}} \times W$$

wherein $SUV_{ROI}$ is a standardized uptake value for a region of interest; $A_{C,ROI}$ is a drug radioactivity concentration in the region of interest; $W$ is a body weight of the irradiated body; $\Delta t$ is a delay between time of drug injection and time of scan start; $T_{\frac{1}{2}}$ is a half-life of a drug's radionuclide; and $D$ is a dose of an injected drug.

7. The method for locating a region of interest in radiation therapy according to claim 1, **characterized in that** the region of interest is defined in the first image data based on the set value.

8. The method for locating a region of interest in radiation therapy according to claim 1, **characterized in that** the second image data is obtained by fusing a second functional image with a second sectional image.

9. The method for locating a region of interest in radiation therapy according to claim 8, **characterized in that** the first image data is registered with data of the second sectional image, and the first image data has a higher definition than the data of the second sectional image.

10. The method for locating a region of interest in radiation therapy according to claim 1, **characterized in that** the first image data is acquired by a first imaging device, and the second image data is acquired by a second imaging device different from the first imaging device.

**11.** The method for locating a region of interest in radiation therapy according to claim 10, **characterized in that** the second imaging device is a multi-mode imaging device.

**12.** The method for locating a region of interest in radiation therapy according to claim 11, **characterized in that** emission computed tomography and computed tomography are used for the multi-mode imaging device.

**13.** A system for locating a region of interest in radiation therapy, **characterized in that** an irradiated body has a first state in which no marker is injected and a second state in which a marker is injected; the system comprising:

an imaging device configured to acquire first image data of the irradiated body in the first state or the second state, and second image data of the irradiated body in the second state; and
a processing device configured to: acquire a reference region based on the first image data or the second image data; obtain a marker parameter based on the second image data, the marker parameter of the reference region being a reference value; and determine a set value based on the reference value and define a region of interest based on the set value.

**14.** The system for locating a region of interest in radiation therapy according to claim 13, **characterized in that** the set value is a ratio of a marker parameter of a region to be defined to the reference value, and the set value is at least 1.5.

**15.** The system for locating a region of interest in radiation therapy according to claim 13, **characterized in that** the set value is a marker parameter of a region to be defined, and the set value is at least 1.5 times the reference value.

Acquire first image data of an irradiated subject in a first state or a second state — S100

Acquire second image data of the irradiated subject in the second state — S200

Acquire a reference region based on the first image data and map the reference region to the second image data — S300

Obtain a marker parameter based on the second image data — S400

Determine a set value based on a reference value and define a region of interest based on the set value — S500

*FIG. 1*

*FIG. 2*

*FIG. 3*

FIG. 4

(1)  (2)  (3)

Drug
accumulation
region

FIG. 5

*FIG. 6 (1)*                    *FIG. 6 (2)*

## INTERNATIONAL SEARCH REPORT

| International application No. |
| --- |
| **PCT/CN2024/088210** |

### A. CLASSIFICATION OF SUBJECT MATTER

A61N 5/10(2006.01)i

According to International Patent Classification (IPC) or to both national classification and IPC

### B. FIELDS SEARCHED

Minimum documentation searched (classification system followed by classification symbols)

IPC: A61N

Documentation searched other than minimum documentation to the extent that such documents are included in the fields searched

Electronic data base consulted during the international search (name of data base and, where practicable, search terms used)

CNABS: CNTXT; VEN; USTXT; EPTXT; WOTXT; CNKI; IEEE: 标记物, 硼中子, 配准, 对位, 融合, 参考区域, 感兴趣区域, 标记摄取值, label, boron neutron, registration, align, fusion, mix, reference area, ROI, SUV

### C. DOCUMENTS CONSIDERED TO BE RELEVANT

| Category* | Citation of document, with indication, where appropriate, of the relevant passages | Relevant to claim No. |
| --- | --- | --- |
| A | CN 109961834 A (SHANGHAI UNITED IMAGING HEALTHCARE CO., LTD.) 02 July 2019 (2019-07-02)<br>description, paragraphs [0006]-[0142] | 1-15 |
| A | CN 102740769 A (KONINKL PHILIPS ELECTRONICS N.V.) 17 October 2012 (2012-10-17)<br>entire document | 1-15 |
| A | CN 115511757 A (GE PRECISION HEALTHCARE, LLC) 23 December 2022 (2022-12-23)<br>entire document | 1-15 |
| A | US 2013136328 A1 (JANSEN FLORIBERTUS HEUKENSFELDT et al.) 30 May 2013 (2013-05-30)<br>entire document | 1-15 |

☐ Further documents are listed in the continuation of Box C.   ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
| --- | --- |
| **06 August 2024** | **12 August 2024** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2024/088210**

| Patent document cited in search report | | | Publication date (day/month/year) | Patent family member(s) | | | Publication date (day/month/year) |
|---|---|---|---|---|---|---|---|
| CN | 109961834 | A | 02 July 2019 | None | | | |
| CN | 102740769 | A | 17 October 2012 | CN | 102740769 | B | 30 September 2015 |
| CN | 115511757 | A | 23 December 2022 | None | | | |
| US | 2013136328 | A1 | 30 May 2013 | None | | | |

Form PCT/ISA/210 (patent family annex) (July 2022)